# EUROPEAN PATENT APPLICATION

(11) **EP 2 259 047 A1**
(43) Date of publication of application: **08.12.2010**
(21) Application number: 09161749.8
(22) Date of filing: 03.06.2009
(51) Int. Cl.: G01N 21/47, A61B 5/00

(54) **Reflectance spectrum measurement at various angles**

(71) Applicant: Koninklijke Philips Electronics N.V., 5621 BA Eindhoven (NL)
(72) Inventor: The designation of the inventor has not yet been filed
(74) Representative: Damen, Daniel Martijn

(57) **Abstract**

Measuring reflectance of skin uses an adjustable probe having an illuminator (100) for directing light of more than one wavelength at a given angle onto the skin, a collector (95, 110, 122, 128) for collecting light reflected from the sample at a given angle, and an adjuster (5, 98, 122, 200, M1, M2), for adjusting the angle of the illuminating light or the collected light. Reflectance is determined according to a predetermined relationship between penetration depth at which the reflections occur, and the wavelength and the angle of adjustment. The angle can be adjusted according to the relationship, or measurements can be selected, so as to maintain a constant penetration depth for a spectrum of wavelengths.

## Description

### FIELD OF THE INVENTION

This invention relates to apparatus for measuring reflections from a sample, e.g., to minimally invasive or non-invasive glucose monitoring apparatus, to corresponding methods, e.g., to minimally invasive or non-invasive glucose monitoring methods, to adjustable probes for measurements in, for example, minimally invasive or non-invasive glucose monitoring, to corresponding methods of controlling such probes, to corresponding methods of measuring reflections, and to computer programs for carrying out such methods.

### BACKGROUND OF THE INVENTION

Many techniques have been investigated to detect skin analyte(s) concentration non-invasively by means of optical, electrical and/or optoelectronic methods, e.g., non-invasive glucose monitoring. Currently a number of companies are developing instruments for non-invasive glucose measurements based on optical methods. A major problem comes from the varying optical properties of human skin tissue. Although these methods are proven to have sufficient sensitivity for in-vitro and/or ex-vivo glucose quantification, none of the currently existing companies was successful in bringing a non-invasive device to the market. The main reason is that the accuracy of recently developed devices is not sufficient to get FDA approval.

Instruments and methods have been developed for minimally invasive measurement of physiological parameters in a human or animal body, such as, for example, glucose measurements based on optical methods. These methods make use of a sensor implanted beneath the skin which is in contact with subcutaneous fluids. The sensor may include gels, particles, liquids which are biodegradable. Preferably, the biosensor that has to be implanted is small in size, and does not require a complicated or painful insertion below the skin.

Non-invasive measurement is the most desirable method for consumers. But the uncertainty and inaccuracy has hampered the acceptance of non-invasive test. There is a need in the non-invasive glucose-monitoring market to solve the inaccuracy or unreliability problems.

Typically, in vivo measurements deal with a larger number of chemical, physical, and physiological interferents in comparison to in vitro case. Information on the presence of interferents, their effects, and variability range are often not known. Typically, data analysis of a system where a number of interferents is present is based on chemometric tools, e.g., multivariate analysis. However, for such a complex and variable system as human tissue, chemometric analysis becomes more complicated and is prone to large errors. The accuracy and reproducibility of these measurements are generally poor due to the many interferents and irreproducibility compared with an in vitro case.

It is known from WO2007100648 to provide a reflectance instrument in which the angle of illumination or angle of collection of the reflection is altered to provide a measurement at various depths beneath the surface of a sample of tissue.

### SUMMARY OF THE INVENTION

An object of the invention is to provide apparatus for measuring reflections from a sample, e.g., minimally invasive or non-invasive glucose monitoring apparatus, to provide corresponding methods, e.g., minimally invasive or non-invasive glucose monitoring methods, to provide adjustable probes for measurements in, for example, minimally invasive or non-invasive glucose monitoring, to provide corresponding methods of controlling such probes, to provide corresponding methods of measuring reflections, or to provide computer programs for carrying out such methods.

According to a first aspect, the invention provides:

Apparatus for measuring reflectance of a sample, the apparatus having an illuminator for directing light of more than one wavelength at a given angle onto the sample, a collector for collecting light reflected from the sample at a given angle, and an adjuster for adjusting the angle of at least one of the illuminating light or the collected light to enable the collected light to represent reflectance at different angles for different wavelengths, and the apparatus being arranged to determine the reflectance from the collected light according to a predetermined relationship of variations in penetration depth in the sample at which the reflections occur for different wavelengths and angles.

By using such a relationship, one notable interferent in the measurements can be reduced or eliminated, or accounted for in any way. That interferent is unwanted variation in penetration depth according to wavelength, which otherwise can contribute to uncertainty about which part of the sample is responsible for some parts of the reflectance. This is particularly useful for measurements of samples having complex structures such as cells or tissue or skin for example, which may give widely different reflections at different penetration depths.

Embodiments of the invention can have any other features added; some such additional features are set out in dependent claims and described in more detail below.

Other aspects of the invention include an adjustable probe for such apparatus, and to methods of controlling such an adjustable probe, to corresponding methods of making measurements, and to computer programs for carrying such methods of controlling or methods of measuring.

In particular the present invention relates to a computer program product that, when executed on a processing engine, implements any of the methods or the apparatus of any of the embodiments of the present invention. The computer program product can be stored on any suitable machine readable signal storage device such as magnetic disk memories such as diskettes or hard drives, optical storage media such as CDROM or DVD ROMs, magnetic tape media or solid state memories such as USB sticks.

Any of the additional features can be combined together and combined with any of the aspects. Other advantages will be apparent to those skilled in the art, especially over other prior art. Numerous variations and modifications can be made without departing from the claims of the present invention. Therefore, it should be clearly understood that the form of the present invention is illustrative only and is not intended to limit the scope of the present invention.

### BRIEF DESCRIPTION OF THE DRAWINGS

How the present invention may be put into effect will now be described by way of example with reference to the appended drawings, in which:
FIG. 1 shows a schematic view of a probe for measuring reflectance showing how a fixed angle leads to sample volumes having different penetration depths at different wavelengths.
FIG. 2 shows a similar view to that of figure 1, and showing the effect of a probe according to an embodiment of the invention, in which the adjusted angles of the illumination fiber and the collection fiber lead to sample volumes of consistent penetration depth for all wavelengths.
FIG. 3 shows an example of results of a Monte Carlo simulation to generate a three dimensional graph showing a relationship between angle, wavelength and penetration depth.
FIG. 4 shows the iso-lines of constant penetration depth as angle varies along the y axis and wavelength varies along the x axis.
FIG. 5 shows a similar graph to that of figure 4 with a highlighted iso-line for one specific penetration depth.
FIGS. 6 and 7 show graphs of calculated changes in angle with time as the wavelength is scanned during a single measurement scan according to an embodiment using the iso-line of figure 5.
FIG. 8 shows a graph of angle with time during a stepwise angle scan according to another embodiment where at each angle a full spectrum is being recorded.
FIG. 9 shows a graph similar to that of figure 5 and showing how to select the correct wavelength parts of one full spectrum scan at a given angle during one part of a stepwise angle scan as in figure 8.
FIG. 10 shows a graph of absorption (which represent reflectance) measurements from one full spectrum scan at a given angle during one part of a stepwise angle scan as in figure 8, highlighting the correct spectral regimes from the full spectrum for a single angle, based on the selections shown in figure 9.
FIG. 11 shows a schematic view of a probe according to an embodiment having steerable mechanical supports.
FIG. 12 shows a schematic view of a probe according to an embodiment having a multi axis support and a motorized slider.
FIG. 13 shows a schematic view of apparatus according to an embodiment.
FIG. 14 shows a schematic view of apparatus according to an embodiment using optical components to adjust the angle or wavelength.

### DESCRIPTION OF THE PREFERRED EMBODIMENTS

The present invention will be described with respect to particular embodiments and with reference to certain drawings but the invention is not limited thereto but only by the claims. The drawings described are only schematic and are non-limiting. In the drawings, the size of some of the elements may be exaggerated and not drawn on scale for illustrative purposes. Where the term "comprising" is used in the present description and claims, it does not exclude other elements or steps. Where an indefinite or definite article is used when referring to a singular noun e.g., "a" or "an", "the", this includes a plural of that noun unless something else is specifically stated.

The term "comprising", used in the claims, should not be interpreted as being restricted to the means listed thereafter; it does not exclude other elements or steps. Thus, the scope of the expression "a device comprising means A and B" should not be limited to devices consisting only of components A and B. It means that with respect to the present invention, the only relevant components of the device are A and B.

Furthermore, the terms first, second, third and the like in the description and in the claims, are used for distinguishing between similar elements and not necessarily for describing a sequential or chronological order. It is to be understood that the terms so used are interchangeable under appropriate circumstances and that the embodiments of the invention described herein are capable of operation in other sequences than described or illustrated herein.

Moreover, the terms top, bottom, over, under and the like in the description and the claims are used for descriptive purposes and not necessarily for describing relative positions. It is to be understood that the terms so used are interchangeable under appropriate circumstances and that the embodiments of the invention described herein are capable of operation in other orientations than described or illustrated herein.

Reference throughout this specification to "one embodiment" or "an embodiment" means that a particular feature, structure or characteristic described in connection with the embodiment is included in at least one embodiment of the present invention. Thus, appearances of the phrases "in one embodiment" or "in an embodiment" in various places throughout this specification are not necessarily all referring to the same embodiment, but may. Furthermore, the particular features, structures or characteristics may be combined in any suitable manner, as would be apparent to one of ordinary skill in the art from this disclosure, in one or more embodiments.

Similarly it should be appreciated that in the description of exemplary embodiments of the invention, various features of the invention are sometimes grouped together in a single embodiment, figure, or description thereof for the purpose of streamlining the disclosure and aiding in the understanding of one or more of the various inventive aspects. This method of disclosure, however, is not to be interpreted as reflecting an intention that the claimed invention requires more features than are expressly recited in each claim. Rather, as the following claims reflect, inventive aspects lie in less than all features of a single foregoing disclosed embodiment. Thus, the claims following the detailed description are hereby expressly incorporated into this detailed description, with each claim standing on its own as a separate embodiment of this invention.

Furthermore, while some embodiments described herein include some but not other features included in other embodiments, combinations of features of different embodiments are meant to be within the scope of the invention, and form different embodiments, as would be understood by those in the art. For example, in the following claims, any of the claimed embodiments can be used in any combination.

Furthermore, some of the embodiments are described herein as a method or combination of elements of a method that can be implemented by a processor of a computer system or by other means of carrying out the function. Thus, a processor with the necessary instructions for carrying out such a method or element of a method forms a means for carrying out the method or element of a method. Furthermore, an element described herein of an apparatus embodiment is an example of a means for carrying out the function performed by the element for the purpose of carrying out the invention. References to a signal can encompass any kind of signal in any medium, and so can encompass an electrical or optical or wireless signal or other signal for example. References to analyzing can encompass processing a signal in any way to derive or enhance information about the material. References to a controller can encompass any means for controlling and so can encompass for example a personal computer, a microprocessor, analog circuitry, application specific integrated circuits, software for the same, and so on.

In the description provided herein, numerous specific details are set forth. However, it is understood that embodiments of the invention may be practiced without these specific details. In other instances, well-known methods, structures and techniques have not been shown in detail in order not to obscure an understanding of this description.

### Introduction to some issues addressed by some of the embodiments

By way of introduction to the embodiments, the problem of interference sources in optical sensing such as non-invasive or minimally invasive blood glucose monitoring will be discussed briefly. The measurement of glucose through near-infrared spectroscopy is based on a change in the concentration of glucose being indicated by a change in the absorption of light according to the absorption and scattering properties of glucose and/or the effect of glucose changes upon the anatomy and physiology of the sampled site. However, in addition to the effect of glucose on the near-infrared light probing signal that is delivered to the skin, the probing signal is also reflected, diffusely reflected, transmitted, scattered, and absorbed in a complex manner related to the structure and composition of the tissue. When near-infrared light is delivered to the skin, a proportion of reflected light, or specular reflectance, is typically between 4-7% of the delivered light over the entire spectrum. Absorption by the various skin constituents accounts for the spectral extinction of the light within each layer. Scattering is the main process by which the beam may be returned to contribute to the diffuse reflectance of the skin. Scattering also has a strong influence on the light that is diffusely transmitted through a portion of the skin.

The scattering of light in tissues is in part due to discontinuities in the refractive indices on the microscopic level, such as the aqueous-lipid membrane interfaces between each tissue compartment or the collagen fibrils within the extracellular matrix. The spectral characteristics of diffuse remittance from tissue result from a complex interplay of the intrinsic absorption and scattering properties of the tissue, the distribution of the heterogeneous scattering components, and the geometry of the point(s) of irradiation relative to the point(s) of light detection.

The near-infrared absorption of light in tissue is primarily due to overtone and combination absorbances of C-H, N-H, and O-H functional groups. As skin is primarily composed of water, protein, and fat; these functional groups dominate the near-IR absorption in tissue. As the main constituent, water dominates the near-infrared absorbance above 1100 nm and is observed through pronounced absorbance bands at 1450, 1900, and 2600 nm. Protein in its various forms, in particular, collagen is a strong absorber of light that irradiates the dermis. Near-infrared light that penetrates to subcutaneous tissue is absorbed primarily by fat. In the absence of scattering, the absorbance of near-infrared light due to a particular analyte, A, can be approximated by Beer's Law. An approximation of the overall absorbance at a particular wavelength is the sum of the individual absorbance of each particular analyte given by Beer's Law. The concentration of a particular analyte, such as glucose, can be determined through a multivariate analysis of the absorbance over a multiplicity of wavelengths because it is unique for each analyte. However, in tissue compartments expected to contain glucose, the concentration of glucose is at least three orders of magnitude less than that of water. Given the known extinction coefficients of water and glucose, the signal targeted for detection by reported approaches to near-infrared measurement of glucose, i.e. the absorbance due to glucose in the tissue, is expected to be, at most, three orders of magnitude less than other interfering tissue constituents. Therefore, the near-infrared measurement of glucose requires a high level of sensitivity over a broad wavelength range. Multivariate analysis is often utilized to enhance sensitivity.

In addition, the diverse scattering characteristics of the skin, e.g., multiple layers and heterogeneity, cause the light returning from an irradiated sample to vary in a highly nonlinear manner with respect to tissue analytes, in particular, glucose. Simple linear models, such as Beer's Law have been reported to be invalid for the dermis.

Dynamic properties of the skin also add to the difficulties. Variations in the physiological state and fluid distribution of tissue profoundly affect the optical properties of tissue layers and compartments over a relatively short period of time. For all these reasons therefore, the optical properties of the tissue sample are modified in a highly nonlinear and profound manner that introduces significant interference into non-invasive tissue measurements.

The measurement of glucose through spectroscopy can also be made by a change in the absorption of light according to the absorption and scattering properties of minimally invasive microsensors, or to the change in light emitted or reflected from such microsensors located below the skin. These methods can suffer all or some of the problems mentioned above. Such methods using microsensors may include, for example,
- observing fluorescence (e.g., fluorescence resonance energy transfer) of a competitive binding assay encapsulated in microcapsules, for example based on competitive binding between the protein Concanavalin A and various saccharide molecules, specifically a glycodendrimer and glucose, the microcapsules can be polyelectrolyte microcapsules,
- detecting glucose using boronic acid-substituted violegens in fluorescent hydrogels in which a fluorescent anionic dye and a viologen are appended to boronic acid, which serve as glucose receptors, and are immobilized into a hydrogel, the fluorescence of the dye being modulated by the quenching efficiency of the viologen based receptor which is dependent upon the glucose concentration,
- other methods, e.g., to monitor oxygen or pH or other "smart tattoo" methods.

### Wavelength dependence

Notably, absorption and scattering in human skin tissue are highly wavelength dependent. This leads to very different pathways of photons inside the measurement volume. Especially in diffuse reflectance optical configuration this is a major issue. In these kinds of methods, light is sent into the skin. In the skin, light is being scattered and absorbed. At a different location than the incidence light, the diffuse reflected light is collected and analyzed. The collected spectrum is representative for the measurement volume that was passed through by the light. However, different absorption and scattering will lead to substantially different pathways for different wavelengths. In turn this leads to not comparable measurement volumes over the complete wavelength regime. Also in minimally invasive techniques using subcutaneously buried microsensors, returning light from the microsensor can take different paths.

### Introduction to features of the embodiments

Embodiments of the invention can have an illuminator for directing light of more than one wavelength at a given angle onto the sample. This can be any kind of illuminator and can encompass a light source or can be for directing light received from an external source. The illuminator can encompass a probe having one or more waveguides such as optical fibers. The fibers can be moved by an actuator to alter the angle, or an optical switch can be used to select which fiber is used, to alter the angle for example.

Embodiments can have a collector for collecting light reflected from the sample at a given angle. Such a collector can encompass a light detector and a probe having one or more waveguides such as optical fibers. The fibers can be moved by an actuator to alter the angle, or an optical switch can be used to select which fiber is used, to alter the angle for example.

Embodiments can have an adjuster for adjusting the angle of at least one of the illuminating light or the collected light. This can encompass any kind of adjuster, such as an actuator as mentioned above for moving the angle of the fiber or fibers relative to the sample, or an optical switch for selecting which of a numbers of fibers at different angles are used.

In embodiments of the apparatus the reflectance can be measured at different angles for one or more different wavelengths and reflectance determined according to a relationship between penetration depth in the sample at which the reflections occur, and wavelengths and angles. This making of measurements according to the relationship can encompass for example selecting only those measurements which the relationship indicates correspond to a given penetration depth. It can also encompass adjusting the angle for each wavelength according to the relationship, so that the measurements are only made at the given penetration depth. Other ways of determining the measurements according to the relationship can be envisaged, for example in some cases the angle could be fixed and the wavelength varied, and only those measurements which the relationship indicates correspond to a given penetration depth would be selected.

An array of measurements at different wavelengths for a given depth can be collected to form a spectrum for that depth. Different spectra at different depths can be gathered for a given sample. Further analysis of any type can be made using the measurements, or the spectra of measurements. Many measurements can be made and averaged to reduce noise. The adjustment of the incidence angle can be made according to a predefined algorithm, which is based on the relationship which may be a relationship of skin optical properties.

Also the present invention also includes microsensors for use with minimally invasive techniques.

### Figure 1

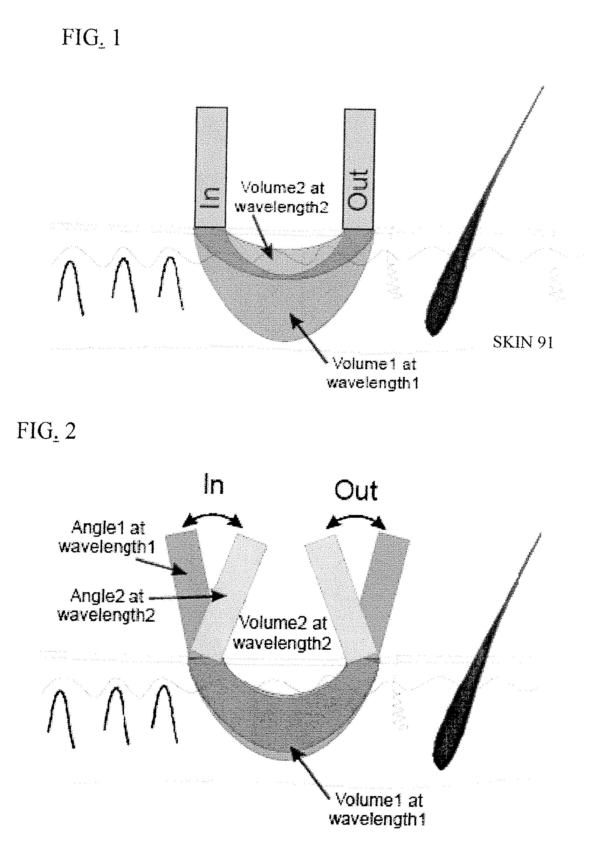

Figure 1 shows a schematic side view of a typical situation without the proposed invention for the sake of comparison. It shows a schematic view of a probe for measuring reflectance showing how a fixed angle leads to sample volumes having different penetration depths at different wavelengths. There is a sample in the form of skin 91 showing various artifacts of the skin at different depths below a surface, such as a hair and other features as would be well known to those skilled in the art. A waveguide such as an optical fiber is shown marked "in" for the illuminating light. Another such waveguide such as an optical fiber is shown marked "out" for the collected light. A volume 1 is shown in the skin extending between the waveguides, shown as a shaded deeply curved area representing the volume in which reflection occurs at a first wavelength. A second volume 2 is shown in the skin extending between the waveguides, shown as a shaded shallower curved area representing the volume in which reflection occurs at a second wavelength. A darker shaded area represents an overlap of the two volumes. As can be seen there is not much overlap, as the ranges of depths of the first and second volumes are largely different.

References to penetration depth can encompass a single value but more typically refers to a volume encompassing a range of depths. The volume may be arc shaped or crescent shaped as shown, and so the range of depths varies with distance from the waveguides.

It is clearly visible in figure 1 that different measurement volumes are being probed when different wavelengths are used, and so measurements of a spectrum of wavelengths will not represent a consistent volume of the sample.

### Figure 2

Figure 2 shows a similar view to that of figure 1, and showing the effect of a probe according to an embodiment of the invention, in which the adjusted angles of the illumination fiber and the collection fiber lead to sample volumes of consistent penetration depth for various wavelengths. In this case each waveguide has two positions (there may be many positions in practice), a first position is at angle 1 shown in darker shading. A second position is at angle 2 shown in lighter shading. In this example both waveguides are moved, in some cases moving just one of the waveguides can be carried out, especially if the non movable waveguide already has a wide angle of illumination or collection. As can be seen, the first and second volumes are almost completely overlapping, meaning that there is a constant penetration depth for the different wavelengths.

This figure shows an example of a probe head in which the angle of the optical fibers is adjustable in such a way, that light will always reach and be reflected from the same sample volume, meaning there is the same penetration depth for different wave lengths.

By adjusting the angle of illumination and collection fiber according to the optical properties of the measured subject it is possible to obtain the same penetration depth for multiple wavelengths in a diffuse reflectance spectroscopy setting. This can result in an increased accuracy and reproducibility of non-invasive or minimally invasive measurement of analytes in skin, e.g., when using diffuse reflectance spectroscopy.

The different optical properties (scattering and absorption) usually lead to different penetration depths in diffuse reflectance spectroscopy. This in turn leads to different measurement volumes for different wavelengths. In summary, this is an unwanted effect in diffuse reflectance spectroscopy, because the measured data is not originating from the same target volume. As described, these issues can be addressed by adjusting the angle of the optical fibers in such a way, that light will always reach the same sample volume or the same penetration depth.

It can also be seen that some embodiments of the invention can be used to maintain the same path length in apparatus for measuring diffuse reflectance.

As will be described in more detail below, some embodiments have a fiber probe setup that allows automatic adjustment of the fiber angle. Some have an algorithm that correlates the wavelength with the desired angle, leading to the same penetration depth. Some have signal processing that selects the correct wavelength at the corresponding angle and that combines the results, to provide a single spectrum at a more constant penetration depth.

### Figure 3

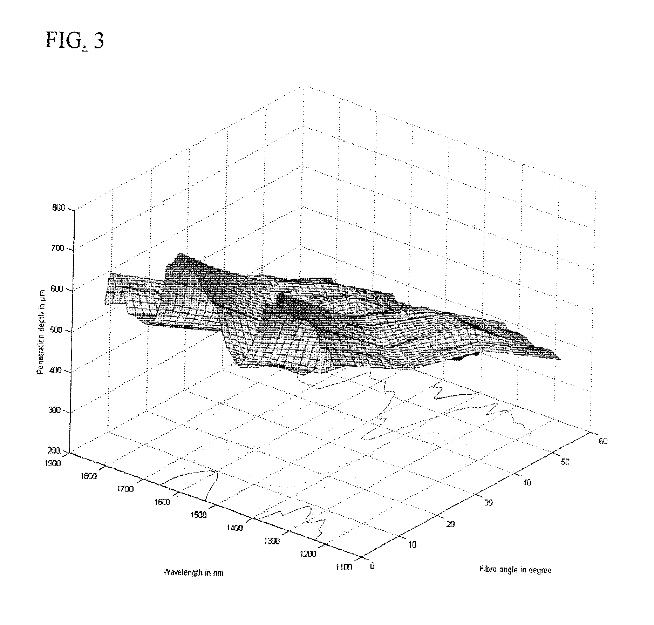

Figure 3 shows a graph of an example of a relationship for use in embodiments. In Figure 3 the floor axes are wavelength and angle, while the height axis shows the corresponding penetration depth. In this case the relationship is for skin, and shows results based on Monte Carlo Photontrace simulations. The relationship can be obtained in any convenient way in principle. The figure shows considerable variations in penetration depth as the wavelength varies and as the incidence angle of the illumination/collection fibers varies. Here a single value for penetration depth has been used, which would in practice represent an average depth of the volumes shown in figures 1 and 2.

### Figures 4 and 5

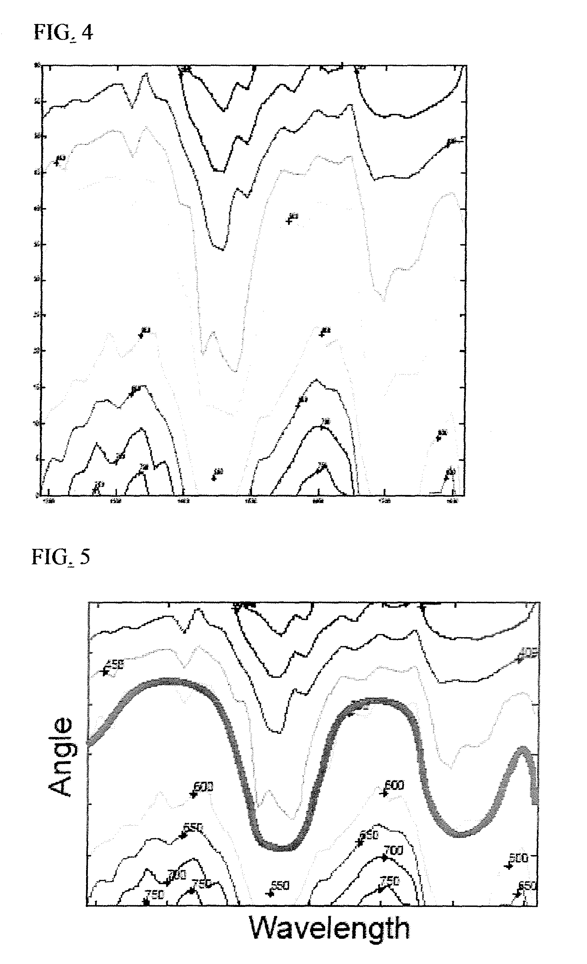

Figure 4 depicts the iso-lines for constant penetration depth in a wavelength vs. angle plot. Following one of these iso-lines leads to constant penetration depth for all wavelengths. This in turn leads to more comparable measurement volume in diffuse reflectance spectroscopy. Figure 5 shows the same plot with a highlighted iso-line for a specific penetration depth. It is clearly visible that an appropriate angle adjustment can be used to enable a constant penetration depth over the complete wavelength regime.

### Figures 6 and 7

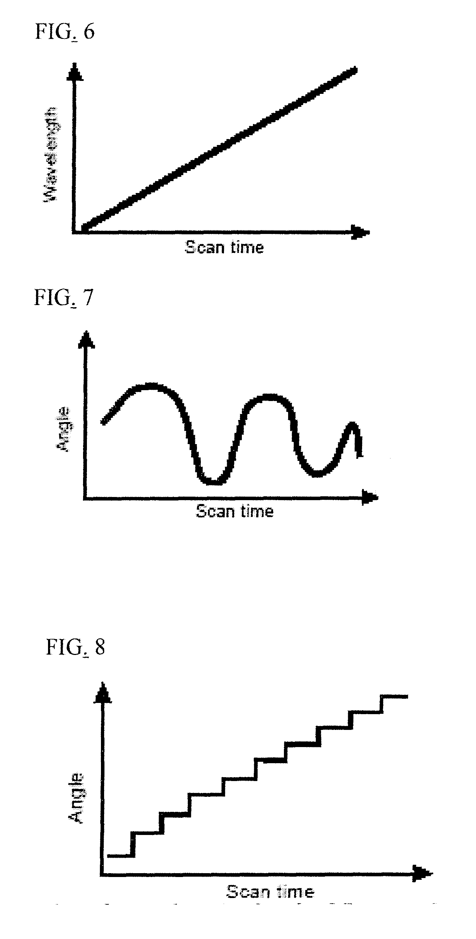

These figures relate to a first embodiment of a method of measuring, which involves adjusting the angle for each wavelength according to the relationship, so that the measurements are only made at the given penetration depth. This can be implemented by scanning the wavelength with time as shown in figure 6, to provide a steadily increasing wavelength. During this scan, the angle is continuously adjusted to follow the iso-line of figure 5 as the wavelength is varied. This is shown in figure 7 and means the penetration depth remains constant and the measurements of the collected light during this scan will represent reflections corrected for depth variations. For corrected penetration depth measurement, the wavelength can be scanned over the whole wavelength region to get a complete spectrum. According to the predefined wavelength/angle correlation, the angle will be adjusted during the wavelength scan. This leads to a linear scan in wavelength and an adjusted scan in angle as shown in Figure 7. This can be implemented by having the relationship stored in a look up table. The current value for wavelength can be fed into the look up table which will output the corresponding angle. This signal can be fed to a device to adjust the angle, such as an actuator such as a motor, or to a selector for selecting from a number of optical signals continuously obtained from different angles, such as a CCD array for example.

Although described above for non-invasive techniques, the same embodiment can be applied for minimally invasive techniques using subcutaneously buried microsensors.

### Figures 8, 9 and 10

These figures relate to a second embodiment involving repeated full wavelength recording at each angle. This embodiment involves a system where the angle is scanned in a linear fashion over the full range of angles. The wavelength is scanned over its full range for each angle, so the angle remains fixed long enough to allow measurements over a full spectrum of wavelengths. Then the angle is altered and the measurements repeated. This stepwise change in angle for each scan is depicted in Figure 8.

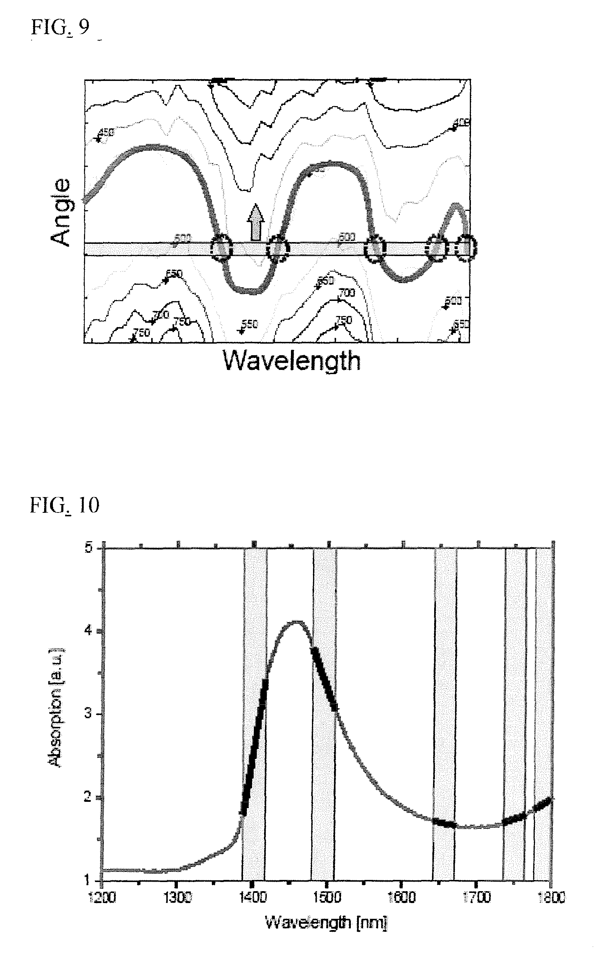

This embodiment uses the relationship not for controlling the adjusting of the angle, but for post processing of the measurements to select the measurements corresponding to certain wavelengths for each angle setting, so that the selected measurements have the same penetration depth. This process is represented in Figure 9 which shows a graph similar to that of figure 5 and showing how to select the correct wavelength parts of one full spectrum scan at a given angle during one part of a stepwise angle scan as in figure 8. The horizontal shaded band represents the measurements taken during one step of the many steps in the scan time shown in figure 8. The iso-line crosses the horizontal band at 5 places which are circled in figure 9. At these five places, the measurements will have a consistent penetration depth. Accordingly these measurements are selected and used to form part of a spectrum.

Figure 10 shows a graph of absorption (which represent reflectance) measurements from one full spectrum scan at a given angle during one step of the many steps shown in figure 8. The selected measurements are shown where the vertical shaded bands cross the line of measurements. This highlights the correct spectral regimes from the full spectrum for a single angle, based on the selections shown in figure 9. At one specific angle, multiple wavelengths may result in measurements at the correct penetration depth. These wavelengths are different for different angles. The post processing part uses the relationship shown in figure 3 or figure 5 to select the relevant measurements from a next scan at a next angle and so on. As each successive angle may produce measurements at the correct depth for different wavelengths, after sufficient angles have been used, there will be sufficient selected measurements at all wavelengths. Hence a complete spectrum can be obtained for a given penetration depth.

In another alternative, the wavelength may be stepped and at each wavelength a complete scan of the range of angles can be carried out. Again, this will result in an array of measurements. From this array of measurements, the desired ones for a given penetration depth, corresponding to the iso-line of figure 5, can be selected.

Although described above for non-invasive techniques, the same embodiments can be applied for minimally invasive techniques using subcutaneously buried microsensors.

### Figures 11 and 12

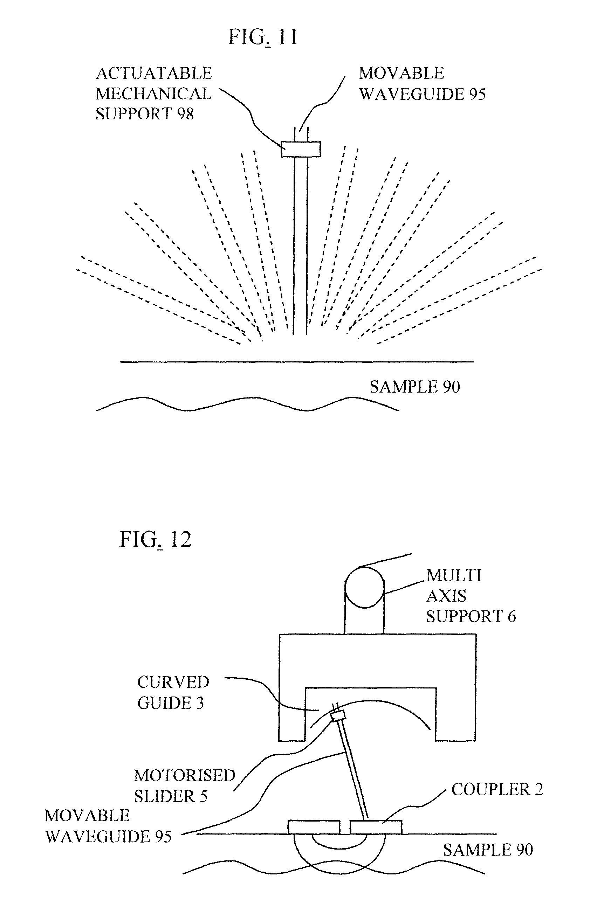

Figure 11 and Figure 12 show possible embodiments how to build the angle adjustment of the fibers. In both cases, two or more beams or fibers can be arranged at various angles with respect to the plane of the tissue surface, and at varying distances relative to one-another. This is achieved by actuation of the beams or fibers, by means of any kind of drive. In both cases the angle of the fibers are adjusted by the drives, which can be electromechanical motors, or hydraulic or other type of actuator as appropriate, together with any kind of gears or other linkage as desired. The fibers will be adjustable about a turning point for the angular adjustment that is close to the tissue surface. The angular movement can be achieved by one motor or by two motors for example.

Figure 11 shows a schematic view of a probe according to an embodiment having steerable mechanical support. This shows a movable waveguide 95 arranged to be moved by an actuatable mechanical support 98. In dotted lines are shown a range of possible positions of the moveable waveguide above a sample 90. The angle of incidence of an optical path of the waveguide changes, but in this example the point of incidence on the surface of the sample remains the same.

Figure 12 shows a schematic view of a probe according to an embodiment having a multi axis support and a motorized slider. In Figure 12, an example of the apparatus comprises an illumination or detection head for mounting an illuminator or collector. In practice both could be mounted on similar supports, only one is shown for clarity. An optical coupler 2 is shown positioned on the outer layer of the sample to be analyzed. The outer layer is for example the skin. Optical coupler is optional and may be a piece of transparent material with a well defined smooth surface. Optical couplers are typically used to correct for the skin roughness so that the relief of the illuminated surface is known when the skin is illuminated. This makes it easier to predict how much light is reflected and how much light penetrates the skin. Optical coupler 2 can also be used with an index matching fluid or gel that is placed between the optical coupler and the skin to prevent any air bubbles being trapped at the interface skin-coupler. The index matching fluid minimizes reflection of light passing through optical coupler and the skin or at the interface between the two. The fluid or gel could also be a type of glue that levels out the skin surface while matching the refractive index of the optical coupler.

The optical coupler may be made out of a light-weight material so that it applies minimal pressure on the skin surface thereby only minimally affecting the physical condition of the lower skin layers of body portion. This can lead to more reliable measurements.

The illumination or detection head is mounted on a multi-axis support 6 which may allow a 6-axis (3 translations, 3 rotations) movement of the head for example. The illumination portion and the detection portion can be mounted to be interdependent, or can be mounted so that the two portions move independently. Also, support 6 may allow less translation and rotational movement, i.e. the support could be a 5 or less -axis type.

The illumination part can comprises a light source such as a bulb or a laser, combined with a reflector to direct the illumination to illuminate the skin area. Conceivably ambient light could be used in suitable environments. A collector part can comprise an optical fiber bundle or a CCD matrix for example. A curved guide 3 is shown for providing a guide for changing the angle of incidence without needing to change the position of the rest of the support. This may adjust the angle of the waveguide 95 using a motorized slider 5. Not shown specifically is the light source or the receiver which may be mounted on the end of the waveguide, or be mounted on the support and coupled to the end of the waveguide by a flexible length of optical fiber for example.

Although described above for non-invasive techniques, the same embodiments can be applied for minimally invasive techniques using subcutaneously buried microsensors.

### Figures 13 and 14

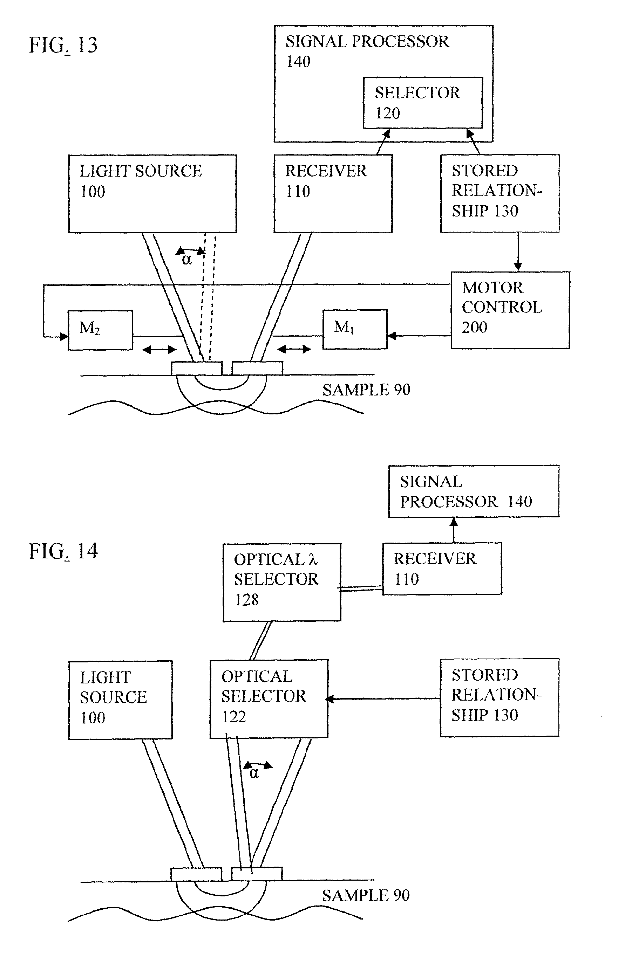

Figures 13 and 14 show schematic views of apparatus according to embodiments. These may be alternatives or may be combined. In figure 13 the angles of the waveguides are adjustable by motors. In figure 14, there are multiple optical waveguides and the angle is adjustable by selecting one of the waveguides. In both cases, selection of wavelength can be carried out at the illuminator side or the collector side. This can be implemented by filters, or by controllable light sources or a combination for example.

In figure 13, a light source 100 is shown coupled by a movable waveguide to a sample 90 through an optical coupler. A motor M2 is shown for moving the waveguide through angle α to a second position marked by dotted lines. A motor M1 is provided for moving a second waveguide coupled to a receiver 110. The second waveguide is also movable though alternative positions are not shown for the sake of clarity. A second position could be envisaged closer to the vertical to correspond to the second position shown in dotted lines for the first waveguide. Also shown is a reflecting volume in the sample between the points of incidence of the optical paths from the waveguides.

A motor control part 200 is shown. This can be implemented by a microprocessor, dedicated circuitry, a PC or any other type of controller. It can also include control software in any conventional computer language following established practice. The motors may be controlled according to a linear scanning algorithm if the method of the second embodiment described above in relation to figs 8, 9 and 10 is used. Alternatively the motors may be controlled according to the relationship of figure 3 as in the first embodiment, described with respect to figs 6 and 7. This is shown in figure 13 by the stored relationship 130 feeding the motor control part. This may be implemented by any kind of store including for example a hard disc of a PC, or by a connection to a remote store over a network link such as the internet.

A signal processor 140 is shown for carrying out the post processing as described above for the second embodiment. This may be implemented by any kind of conventional processing hardware microprocessor, dedicated circuitry, a PC or any other type. This signal processor involves a selector part 120 which is coupled to the stored relationship and may be implemented in software in any conventional computer language following established practice. The control or selection according to the relationship need not involve an explicit look up table. The relationship can be used indirectly or implicitly. For example it can be embedded in a control algorithm, or can be derived in real time from other properties or measurements in principle.

FIG. 14 shows a schematic view of apparatus according to an embodiment using optical components to adjust the angle or wavelength. An optical selector 122 is provided coupled to multiple waveguides set at different angles as a light collector. A similar arrangement can optionally be provided for the illuminator (only shown on the collector side for the sake of clarity). In this way, each beam or fiber can be addressed individually, and the user can select any pair for illumination and collection, appropriate to the application. This selection of fiber and hence angle can be controlled according to a linear scanning algorithm if the method of the second embodiment described above in relation to figs 8, 9 and 10 is used. Alternatively the angle may be controlled according to the relationship of figure 3 as in the first embodiment, described with respect to figs 6 and 7. Again the relationship can be used indirectly or implicitly. For example it can be embedded in a selection control algorithm, or can be derived in real time from other properties or measurements in principle.

An optical wavelength selector 128 is provided for selecting wavelength, for example by providing adjustable optical filters anywhere in the optical path from the light source. This part feeds a receiver 110 which converts the measurements from optical signals into electrical signals. These can be processed typically in the digital domain by a signal processor 140.

As in figure 13, on the illuminator side a light source 100 is shown coupled by a waveguide to a sample 90 through an optical coupler. The waveguide may be movable or fixed, or there may be multiple waveguides provided with a selector.

Although described above for non-invasive techniques, the same embodiment can be applied for minimally invasive techniques using subcutaneously buried microsensors.

### Application

An opto-mechanical probe skin interface with angular adjustment, as proposed here, can be used by most techniques that sense analytes within the skin. Embodiments of the invention can be used for non-invasive glucose detection by means of NIR diffuse back-reflectance spectroscopy. Further applications include measurements of skin properties (e.g., skin cancer, skin aging, etc.) by means of light. Also, an opto-mechanical probe skin interface with angular adjustment, as proposed here can be used for minimally invasive glucose detection as can NIR diffuse back-reflectance spectroscopy.

Other variations can be envisaged within the scope of the claims.

## Claims

1. Apparatus for measuring reflectance of a sample, the apparatus having an illuminator (95, 100) for directing light of more than one wavelength at a given angle onto the sample, a collector (95, 110, 122, 128) for collecting light reflected from the sample at a given angle, and an adjuster (5, 98, 122, M1, M2, 200) for adjusting the angle of at least one of the illuminating light or the collected light relative to the sample, to enable measurements at different angles for different wavelengths, the apparatus being arranged to determine the reflectance from the measurements and according to a predetermined relationship between penetration depth in the sample at which the reflections occur, and the wavelength and the angle of adjustment.

2. The apparatus of claim 1, the arrangement to determine the reflectance according to the relationship comprising a selector (120, 122) to select measurements which correspond to a given penetration depth according to the relationship.

3. The apparatus of claim 1 or 2, the arrangement to determine the reflectance according to the relationship comprising the adjuster (200, M1, M2) which is arranged to adjust the angle of at least one of the illuminating light or the collected light according to the relationship.

4. The apparatus of claim 3, the adjuster being arranged to adjust the angle of at least one of the illuminating light or the collected light for different wavelengths so as to maintain a constant penetration depth.

5. The apparatus of any preceding claim, the adjuster having a movable waveguide (95) and an actuator (98) for moving the movable waveguide to change an angle of incidence of an optical path of the waveguide relative to the sample.

6. The apparatus of any preceding claim, the adjuster being arranged to adjust both the angle of the illuminating light and the angle of the collected light.

7. The apparatus of any preceding claim, arranged to generate an array of measurements over a range of wavelengths and a range of angles.

8. The apparatus of any preceding claim suitable for minimally invasive or non invasive glucose measurements on skin.

9. A method of measuring reflectance of a sample, the method having the steps of directing light of more than one wavelength at a given angle onto the sample, collecting light reflected from the sample at a given angle, adjusting the angle of at least one of the illuminating light or the collected light relative to the sample, to enable measurements at different angles for different wavelengths, and determining the reflectance from the measurements and according to a predetermined relationship between penetration depth in the sample at which the reflections occur, and the wavelength and the angle of adjustment.

10. The method of claim 9, the step of determining the reflectance according to the relationship comprising selecting measurements which correspond to a given penetration depth according to the relationship.

11. The method of claim 9 or 10, the step of determining the reflectance according to the relationship comprising adjusting the angle of at least one of the illuminating light or the collected light according to the relationship.

12. The method of claim 11, the step of adjusting comprising adjusting the angle of at least one of the illuminating light or the collected light for different wavelengths so as to maintain a constant penetration depth.

13. An adjustable probe for use in measuring reflectance of a sample, the probe having an illuminator (100) for directing light of more than one wavelength at a given angle onto the sample, a collector (95, 110, 122, 128) for collecting light reflected from the sample at a given angle, and an adjuster (5, 98, 122, 200, M1, M2) for adjusting the angle of at least one of the illuminating light or the collected light relative to the sample, to enable measurements at different angles for different wavelengths, the adjuster being arranged to adjust the angle according to a predetermined relationship between penetration depth in the sample at which the reflections occur, and the wavelength and the angle of adjustment.

14. The adjustable probe of claim 13, being arranged to adjust the angle so as to maintain a constant penetration depth for different wavelengths.

15. A computer program product that, when executed on a processing engine, implements any of the methods of claims 9 to 12 or the apparatus of any of the claims 1 to 8.
